# EUROPEAN PATENT APPLICATION

(11) **EP 2 840 128 A1**
(43) Date of publication of application: **25.02.2015**
(21) Application number: 13382332.8
(22) Date of filing: 19.08.2013
(51) Int. Cl.: C12M 1/00, C12M 1/42

(54) **Photobioreactor, process and system for algae growth**

(71) Applicant: Llado Contijoch, Francesc, 08232 Viladecavalls (ES)
(72) Inventor: Llado Contijoch, Francesc, 08232 Viladecavalls (ES)
(74) Representative: Sugrañes Patentes y Marcas

(57) **Abstract**

Photobioreactor (PBR) process and system for algae growth, the photobioreactor comprising a tank (2) having an external wall (3), an inlet (4) for adding a medium for algae growth, an outlet (5) for extracting algae from the photobioreactor, CO2 applying means (6) and lighting means (7) further comprising an internal wall (8), determining in the tank a first chamber (9a) and a second chamber (9b) intercommunicated, such that the medium of the tank can flow between chambers; the CO2 applying means being arranged in said first chamber for bubbling CO2 to the medium and alternatively comprising pressure wave pulsation means.. The system (1) also comprises a water preparation unit and a nutrition algae system. The water preparation unit comprises means for activating water through cavitation to obtain the prepared water.

## Description

### Technical field of the invention

The present invention is related to a photobioreactor, a process and a system for algae growth, said algae being used for later obtaining fat acids, combustible gas and/or biomass.

### Background of the Invention

Photobioreactors for algae growth are known in the state of the art for growing an algae starting in a tank, starting from a small algae strain in a medium with nutrients until the algae population grows enough to saturate the tank. At this stage, algae can be processes to obtain products such as biodiesel, biomass and fat acids. Given the wide range of products that can be obtained from algae, it is desirable the growth of the algae population to be as fast as possible. For example WO201045631 discloses a method and unit for algal biomass production, for growing and sequentially processing algae to produce a variety of products such as biofuels, algal meal using a small reactor and a bioreactor.

In order to increase the growth ratio of the algae, photobioreactors like the one disclosed in the patent document WO201144194 are known, which uses an emulsion comprising algae and a gaseous stream so the nutrients and algae are more homogenously mixed before its introduction to the tank of the photobioreactors.

However, it is desirable having a more efficient photobioreactor allowing a faster growth of algae, so algae production can be increased without the need to use larger photobioreactors.

It is then an objective of the present invention disclosing a photobioreactor, together with a system and a process using said photobioreactor, that increases the algae growth rate compared with the photobioreactors known in the state of the art.

### Description of the invention

The photobioreactor for algae growth of the present invention comprises a tank having an external wall, an inlet for adding a medium for algae growth formed by a solution comprising an algae strain, such as Chlorella, to be grown in the photobioreactor, prepared water and a nutrition solution to the tank. The photobioreactor also comprises an outlet for extracting algae from the photobioreactor, CO2 applying means and lighting means.

In essence, the photobioreactor is **characterised in that** it further comprises an internal wall, determining a first chamber and a second chamber in the tank, said first and second chambers being intercommunicated, such that the medium of the tank can flow between chambers and the CO2 applying means being arranged in said first chamber for bubbling CO2 to the medium. The first and second chambers can be intercommunicated through at least a top aperture and at least a bottom aperture in the internal wall, such that the medium of the tank can change chambers through said top and bottom apertures and the CO2 applying means being arranged in said first chamber for bubbling CO2 in a vertical direction to the medium, so the medium of said first chamber is directed upwards, passing to the second chamber through the top apertures, and being directed downward in said second chamber and returning to the first chamber through the bottom apertures, in a circular motion, allowing the algae to move in the tank and mix homogenously with the CO2 and nutrients of the medium.

In a variant of the invention, the internal wall is cylindrical, the first chamber being surrounded by said internal wall and the second chamber being determined between the internal wall and the external wall, increasing the homogenous mix of the algae with the CO2 and nutrients of the medium.

In another variant of the invention, said first chamber further comprises a pressure wave pulsation means, for applying pressure waves in a vertical direction, said pressure wave pulsation means being placed behind the CO2 applying means, such that the pressure waves generated by the pressure wave pulsation means hit the CO2 bubbled by the CO2 applying means, increasing the absorption of nutrients and CO2 by the algae. When the internal wall is cylindrical it allows the propagation of the pressure waves in a vertical direction through the first chamber, pushing the content solution towards the second chamber through the top apertures and the return of said content to the first chamber though the bottom apertures.

In a variant of the invention, the pressure wave pulsation means are placed at the bottom of the first chamber, arranged to generate vertical pressure waves in said first chamber, advantageously hitting the content solution containing the algae through all the length of the first chamber. It is advisable to arrange the pressure wave pulsation means such that the shape of the pressure wave coincide with the section of the first chamber to minimise or even avoid turbulences near in the walls of said first chamber.

In a variant of the invention, the CO2 applying means is an essentially flat tubular spiral provided with holes for bubbling CO2, evenly distributing the bubbled CO2 among the section of the first chamber and allowing the medium to pass across it, allowing to circulate in a circular motion.

In a variant of the invention the CO2 applying means is a fan provided with holes in the lateral edge of its blades for bubbling CO2, advantageously forcing an upward movement of the content solution of the first chamber when being rotated by the action of the CO2 bubbled from its blades.

In a variant of the invention, that the pressure wave pulsation means generates pressure waves with a frequency up to 100MHz and amplitude up to 80mm, which increases the algae metabolism of the algae contained in the content solution. This increase of algae metabolism is obtained by the combination of two effects. The fist effect is that pressure waves impacting on the algae cells surface help cleaning the algae surface. The second effect is that the nutrition elements are pushed when being hit by the pressure waves, thus said nutrition elements being pumped from the first to the second chamber and back in a circular motion. This second effect happens in a higher extent as long as the frequency of the pressure waves gets closer to the resonance frequency of the chemical compounds solved in the solution. It has been noted that when the pressure wave pulsation means generates pressure waves with a frequency between 60Hz and 80Hz and an amplitude between 60mm and 80mm said pressure waves are closer to most of the resonance frequencies of the chemical compounds solved, thus having an optimal pumping effect in this range.

In a variant of the invention, the pressure wave pulsation means comprises a flexible membrane (20) placed at the bottom of the first chamber (9a) and actuating means actuating said flexible membrane to transmit pressure waves to said first chamber.

In a variant of the invention, the photobioreactor further comprises a top membrane placed under the flexible membrane and a bottom membrane, the gap between the top and bottom membranes being surrounded by a coil and said bottom membrane being hydraulically connected to an external flexible membrane placed at the bottom of the second chamber and arranged to generate vertical direction pressure waves in said second chamber.

In a variant of the invention, the gap between the top and bottom membranes contains a ferrofluid that could act as the magnetic body of the actuating means or could be just used to reduce friction. Alternatively, when used to reduce friction, the ferrofluid could be replaced with other any technical liquid or oil.

In a variant of the invention, the gap between the top and bottom membranes contains a shaft attached to the top and bottom membranes rigidly attached to a magnetic body and performing an up and down movement when said coil is powered with an alternate current.

In a variant of the invention, the internal wall is transparent and embeds the lighting means, so light can be applied simultaneously to the fist and second chambers.

In a variant of the invention, the lighting means embedded in the internal wall comprises a glowing liquid placed inside of the internal wall, so light can be evenly distributed through the otobioreactor as glowing liquid radiates omnidirectionally.

In a variant of the invention, the lighting means further comprises a light source powering the glowing liquid and pumping means for moving said glowing liquid within the internal wall, so the glowing liquid can be easily mixed and distributed through all the fotobioreactor. Also, in a variant of the invention, the lighting means placed inside the internal wall comprise led strips which provides light to the photobioreactor in a thin space.

Activated water through cavitation can be used for growing algae in the photobioreactor of the present invention, activated water through cavitation allowing a better dissolution of the elements and nutrients in the water as well as having a lower PH, more acid, than plain water.

The system of the present invention for algae growth comprises a water preparation unit comprising means for filtering said water; means for sterilizing said water; means for carbonating said water.

The system also comprises a nutrition algae system comprising means for mixing Ca(OH)2 with CO2 obtaining a first mix comprising Ca(HCO3)2; means for mixing said first mix with a nitrogen oxide (N2O, NO, NO2) and/or with a sulphur oxide (SO2) to obtain a second mix respectively comprising CaSO4 and/or CaNO4; and means for mixing said second mix with sewage, mainly organic waste, and yeast obtaining prepared nutrition solution.

In essence, the system is **characterised in that** it further comprises one or more photobioreactors; said one or more photobioreactors comprising at least an algae strain to be grown, and being connected to said water preparation unit, for providing prepared water and to said nutrition algae system, for providing nutrition solution to the said one or more photobioreactors; said one or more photobioreactors further comprising lighting means and CO2 applying means to promote the algae population grow in said one or more photobioreactors, and algae processing means connected to said one or more for photobioreactors for processing the algae population and obtaining fat acids, combustible, gas and/or biomass. The water preparation unit of the system further comprises means for activating water through cavitation to obtain prepared water to be used in the system for the algae growth.

In a variant of the invention, the system further comprises a grow reactor comprising an algae strain, connected to said water preparation unit and said nutrition algae system for mixing prepared water and prepared nutrition solution in the grow reactor; the grow reactor being connected to the one or more photobioreactors for transferring at least part of the algae strain.

The process for algae growth comprises the steps of preparing water in a water preparation unit through the sequential steps of filtering said water, sterilizing said water and carbonating said water with the addition of CO2.

The process also comprises the steps of preparing a nutrition solution in a nutrition algae system (NAS) through the sequential steps of mixing Ca(OH)2 with CO2 obtaining a first mix comprising Ca(HCO3)2; mixing said first mix with a nitrogen oxide (N2O, NO, NO2) and/or with a sulphur oxide (SO2) to obtain a second mix respectively comprising CaSO4 and/or CaNO4; and mixing said second mix with sewage and yeast obtaining prepared nutrition solution.

In essence, the process is **characterised in that** it further comprises the step of adding an algae strain to one or more photobioreactors, together with prepared water from the water preparation unit and nutrition solution from the prepared nutrition solution, for growing the algae population, applying light, CO2 and pressure waves for growing the algae population, and processing said algae population from said one or more photobioreactors for obtaining fat acids, combustible, gas and/or biomass. Preparing water in the water preparation unit also comprises the additional step of activating said water through cavitation. Cavitation activated water used in the water preparation unit allows almost full dissolution of all nutrition components for algae growing in said water. It further changes the water PH factor, thus surprisingly increasing the algae absorption ratio of the nutrition components solved in the water, leading to a better algae growth.

In a variant of the invention, the process further comprises the step of mixing prepared water and prepared nutrition solution in a grow reactor comprising an algae strain until the algae population grows before transferring said algae strain to the one or more photobioreactors.

### Brief description of the drawings

Variants of the process and system for obtaining fat acids, combustible gas and/or biomass from algae using at least a photobioreactor are illustrated by way of non-limiting example in the attached drawings. Specifically:
Fig. 1 is a diagram of the preparation of water in the water preparation unit;
Fig. 2 is a diagram of the preparation of the nutrition solution in the nutrition algae system;
Fig. 3 is a diagram of the system for obtaining fat acids, combustible gas and/or biomass from algae of the present invention using photobioreactors;
Fig. 4 shows a photobioreactor of the system;
Fig. 4a shows another variant of the photobioreactor of the system;
Figs. 5a, 5b and 5c show variants of the photobioreactors;
Figs 6, 7, and 8a and 8b shows the base of a variant of the photobioreactor; and
Figs. 9a and 9b shows a variant of the CO2 applying means.

### Detailed description of the drawings

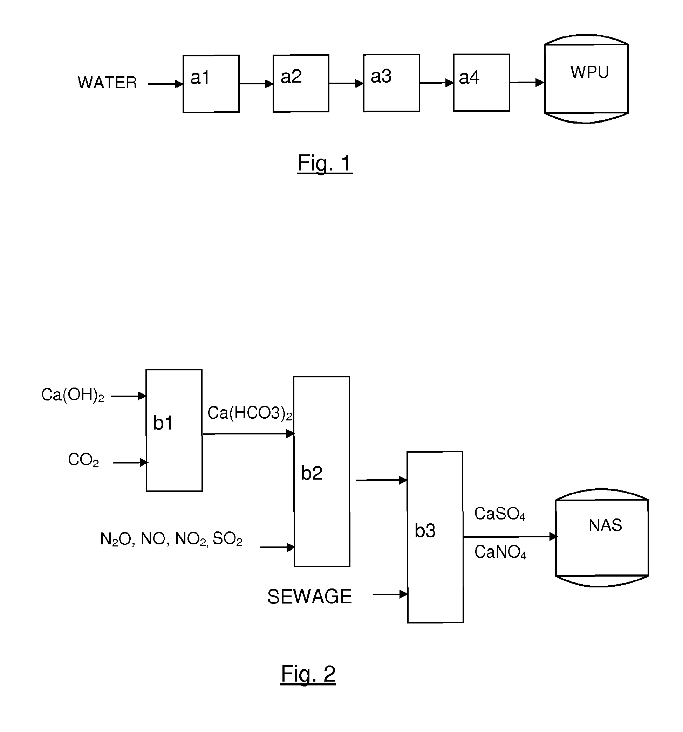

Fig. 1 shows the steps of preparing water in a water preparation unit WPU through the sequential steps of filtering a1 said water for extracting its large fraction, sterilizing a2 said water through a UV sterilizer to kill possible bacteria of said water, carbonating a3 said water with the addition of CO2 and cavitation activating a4 said water for example using a cavitational device to obtain prepared water which will be stored in said water preparation unit WPU.

Advantageously, cavitation activating water will allow almost full dissolution of all nutrition components for algae growing in the cavitation activated water. It further changes the water PH factor, in result, it surprisingly increases the algae absorption ratio of the nutrition components solved in the water, leading to a better algae growth.

Cavitation activation of water is performed using a high speed agitator in which bubbles are created when water flows through said agitator, creating areas with very high pressure gradients in which vacuum bubbles are created. When said vacuum bubbles collapse, shock waves are generated, affecting the molecules and atomic ties of chemical elements solved in the water. This causes ionization of the water and changes the chemical composition of the solved salts in the water. At this stage, water is activated. In result of the activation of water by cavitation, a change on the molecule ties occurs increasing the dissolution elements in water. This also decreases the PH of the water, thus becoming more acid.

Fig. 2 shows the steps of preparing a nutrition solution in a nutrition algae system NAS through the sequential steps of mixing Ca(OH)2 with CO2 obtaining a first mix comprising Ca(HCO3)2; mixing said first mix with a nitrogen oxide (N2O, NO, NO2) and/or with a sulphur oxide (SO2) to obtain a second mix respectively comprising CaSO4 and/or CaNO4; mixing said second mix with sewage and yeast obtaining prepared nutrition solution.

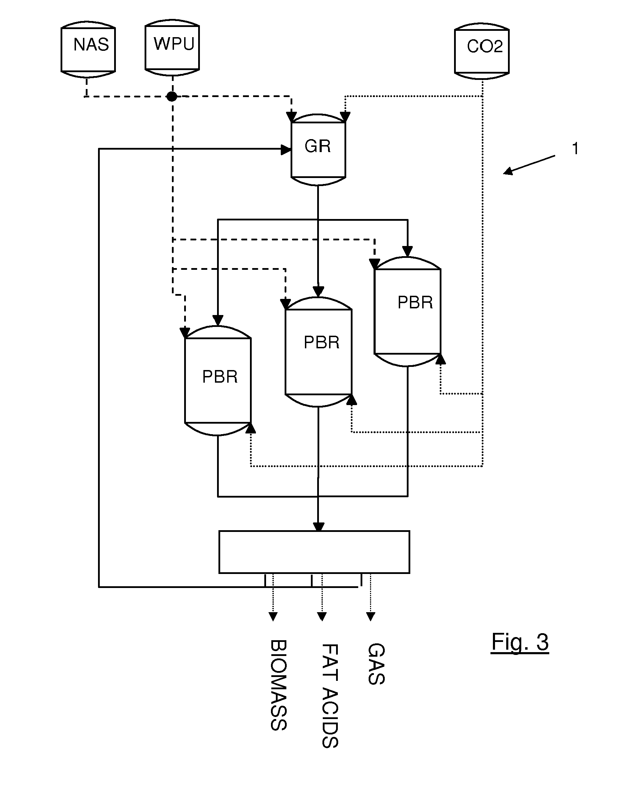

The water preparation unit WPU, containing prepared water, and the nutrition algae system NAS, containing nutrition solution are mixed in a grow reactor GR as depicted in Fig. 3, said grow reactor GR contains at least an algae strain, for example Chlorella but any other suitable strain of algae could be used, that will grow until the algae population reaches a first predetermined value, that could be for example a saturation value in which no more algae can be created, so the algae population stops growing.

At this state, at least part of the algae from the grow reactor GR passes to one or more photobioreactors PBR, in case of the variant shown in Fig. 3, the system 1 comprises three photobioreactors PBR, together with prepared water from the water preparation unit WPU and nutrition solution from the prepared nutrition solution NAS, for growing the algae population in said photobioreactors PBR. If necessary, the grow reactor GR could be removed from the system 1, directly applying the algae with the prepared water from the water preparation unit WPU and nutrition solution from the prepared nutrition solution NAS, however grow reactor GR allows an initial growth of the algae that should be done externally otherwise. Also, different algae strains could be used in each photobioreactor PBR.

Also a pressure wave pulsation is applied to the solution comprising algae of each photobioreactor PBR, said pressure wave pulsation having a frequency between 1 Hz and 100MHz and an amplitude up to 80mm, which increases the absorption of the nutrition solution and the dissolution of CO2 by the algae.

It has been surprisingly seen that said pulsations from the pressure waves increases the algae metabolism. Pulsations created in the liquid impact on algae cells surface, helping to clean the algae surface and also activating the transport of nutrition elements to the algae surface. Although the form and frequency pulsations waves can be variable and different, it has been seen that when the pulsations from the pressure waves happens to be the same as the resonance frequencies of the chemical compounds solved, said chemical compounds are pushed upward, so the chemical compounds are pushed along the whole photobioreactor PBR, reaching all algae contained in the photobioreactor PBR. Therefore, for the mass transfer of food to the algae high pressure of the pressure wave pulsation is desirable. Also high pressure is good for cleaning the cell surface of the algae.

The best absorption of the nutrition solution and the dissolution of CO2 by the algae has been observed for a frequency of 60-80Hz and an amplitude of 60-80 mm. The optimal amplitude will depend on the diameter of the chamber where said pressure wave is created, to create an impact effect inside said chamber, as explained later.

For higher frequencies, i.e. between 80Hz and 100MHz the absorption of the nutrition solution and the dissolution of CO2 by the algae, although not optimal, still makes a difference when compared with the absorption without applying pressure waves.

However, it has been noted that when using pressure waves with frequencies over 100 MHz, said pressure waves have no effect on the absorption of the nutrition solution and the dissolution of CO2 by the algae.

Once the algae strain of the photobioreactors PBR has grown, the algae can be extracted from the photobioreactors PBR through their outlets 5 and the processing the algae for obtaining different final products in known ways. Algae from the photobioreactor PBR can be used directly as forage or additives for feeding cattle; also it can be used for manufacturing alcohol to be used in the cosmetic industry. Algae can also be used as biomass, that can be directly used as a combustible after being completely dried.

If the algae strain used has a high percentage of fat acids, natural gas can also be obtained, for example combining it with methane and catalysts for obtaining natural gas and fertilizers after a fermentation process. Biofuel can also be obtained from the fat acids of the algae as known by the skilled person. Such fat acids can be extracted by mechanical means from the water and the remains of the algae through known means, for example a centrifuge.

After having extracted said fat acids, the water and remains of algae and yeast left, can be returned to the grow reactor GR as they would be fed to the next strain of algae growing in the grow reactor GR, or it can even be used as an additive for animal food.

The process for growing algae in the system 1 using one or more photobioreactos PBR can be automated using a plurality of sensors and actuators, for example valves, light, etc., that can be controlled by a central computer, so the process can be carried out automatically, in an unmanned way.

Alternatively, cavitation activated water can also be used for growing algae in the photobioreactors known in the state of the art. In this case, cavitation activated water can be used instead of the plain water used in said photobioreactors, or cavitation activated water can be mixed with said plain water. An increase of the algae growth rate has been observed when using cavitation activated water for growing algae, due to the previously explained properties of the cavitation activated water independently of the photobioreactor used.

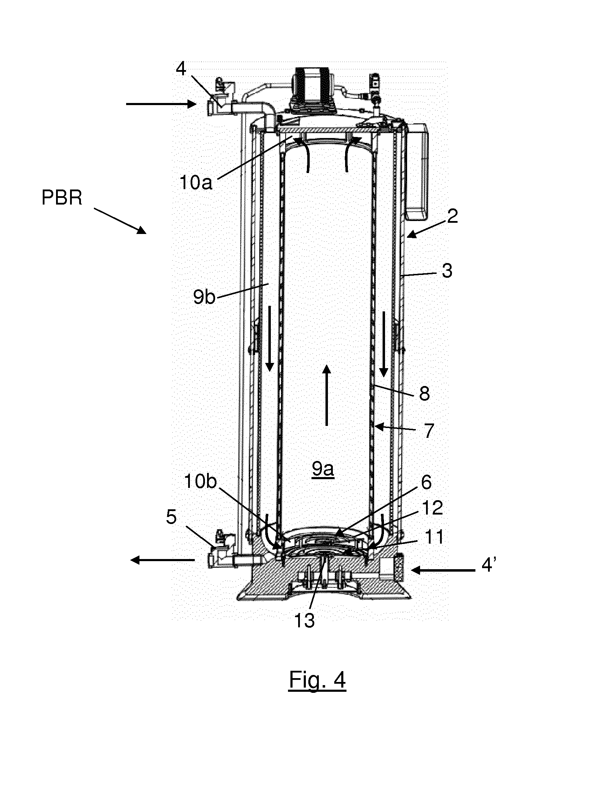

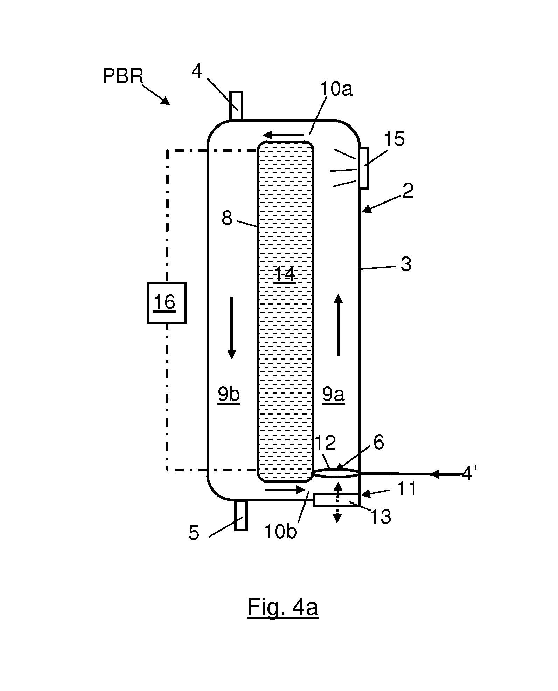

Fig. 4 shows in detail the internal and external parts of one photobioreactor PBR according to the present invention. As it can be seen in Fig. 4 the photobioreactor PBR for algae growth, comprises a tank 2, essentially vertical, that has an external wall 3, an inlet 4 for adding a solution comprising algae, prepared water WPS and nutrition solution NAS to the tank, an outlet 5 for extracting algae from the photobioreactor, CO2 applying means 6 and lighting means 7.

The internal wall 8, determines a first chamber 9a and a second chamber 9b in the tank 2, and said first and second chambers 9a, 9b are intercommunicated through at least a top aperture 10a or passage and at least a bottom aperture 10b or passage in said internal wall, such that the content solution of the tank 2 can change chambers through said top and bottom apertures 10a, 10b, thus recirculating, the CO2 applying means 6 being arranged in said first chamber 9a for bubbling CO2 in a vertical direction, so the medium, the content solution, of said first chamber 9a is directed upwards, as indicated by the arrow, passing to the second chamber 9b through the top apertures 10a, and being directed downward, as indicated by the arrow, in said second chamber 9b and returning so the first chamber 9a through the bottom apertures 10b, in a circular motion.

The first chamber 9a further comprises pressure wave pulsation means 11, for applying pressure waves in a vertical direction, said pressure wave pulsation means 11 are placed behind the CO2 applying means 6, such that the pressure waves generated by the pressure wave pulsation means 11 hit the CO2 bubbled by the CO2 applying means, so the waves are directed in the same direction as the flow of CO2 bubbles, thus hitting said bubbles and allowing a better and most homogeneous mix between the CO2 and the solution of the tank 2.

In the variant of the photobioreactor PBR of Fig. 4 the internal wall 8 is cylindrical, and the first chamber 9a is surrounded by said internal wall 8 and the second chamber 9b being determined between the internal wall 8 and the external wall 3, so the external wall 3 and internal wall 8 are cylindrical and concentric. Naturally, other shapes of the external wall 3 and internal wall 8 could be used, as long as circulation of the algae solution is allowed, for example as in the photobioreactor PBR shown in the Fig. 4a, in which both first chamber 9a and second chamber 9b are separated only in one of their sides by the internal wall 8.

In order to allow the passage of the solution, the CO2 applying means 6 can be arranged as an essentially flat tubular spiral 12 provided with holes for bubbling a mix of air and CO2 fed through a CO2 inlet 4' of the photobioreactor PBR. Alternatively, CO2 can be previously mixed with air in a compressor and later added to the tubular spiral 12. After bubbling CO2, it can be recovered from the top of the photobioreactor PBR and reinjected again to the photobioreactor PBR in a closed circuit.

The photobioreactors PBR of Figs. 4 and 4a have pressure wave pulsation means 11 that are placed at the bottom of the first chamber 9a, the pressure wave pulsation means 11 being arranged to generate pressure waves that propagate in a vertical direction in said first chamber, said longitudinal pressure waves being compression waves travelling upward the first chamber 9a.

Said longitudinal pressure waves can have an amplitude up to 80 mm and frequencies from 1 Hz to 100 Mhz, so the pressure wave pulsation means 11 of the photobioreactor PBR can be a piston 13 capable to generate pressure waves with a frequency up to 100MHz and amplitude up to 80mm. The piston 13 can be mechanically or hydraulically actuated. The pressure wave pulsation means 11 of the photobioreactor PBR could also be any other actuator capable of creating a pressure wave in the previous range of frequencies and amplitudes, for example an electromechanical film or a vibrating membrane.

The internal wall 8 of the photobioreactor PBR is transparent and embeds the lighting means 9. The term transparent can be construed as allowing the passage of the wavelength emitted by the lighting means 9 that promotes the growth of the algae of the photobioreactor PBR.

It can be seen that in the embodiment of Fig. 4a the lighting means 9 of the photobioreactor PBR are embedded in the internal wall 8 and comprises a glowing liquid 14 placed inside the said internal wall 8. The lighting means 9 shown in Fig. 4a further comprises a light source 15 powering the glowing liquid and pumping means 16 for moving said glowing liquid within the internal wall 8.

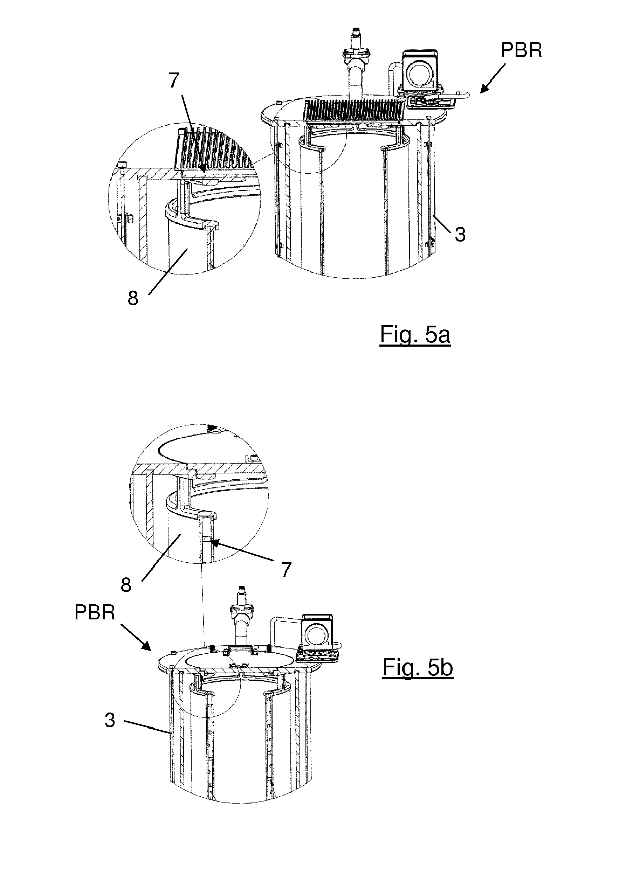

Fig. 5a shows the detail of a variant of a photobioreactor PBR in which the lighting means 9 is a surface provided with leds facing towards the interior of the photobioreactor PBR,

Fig. 5b shows a variant of a photobioreactor PBR in which the lighting means 9 are strips of leds placed inside the inner wall, such that the emitted light reaches the first and second chamber.

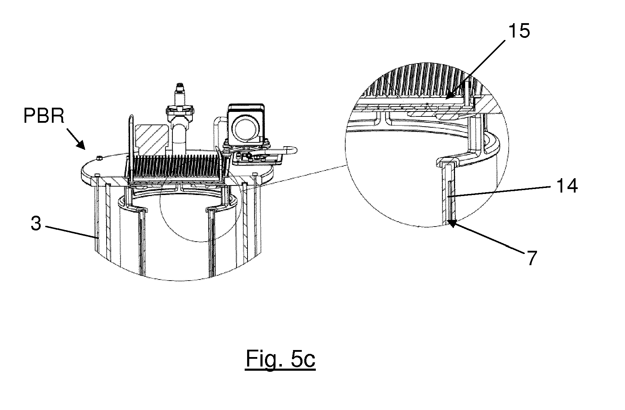

Fig. 5c shows a variant of a photobioreactor PBR in which lighting means 9 are embedded in the internal wall 8 and comprises a glowing liquid 14 placed inside of the internal wall 8. Said glowing liquid, can be for example a fluorescent o fosforescent liquid or any other liquid capable of absorbing light or any other radiation and being able to emit light, in the same or different wavelength later on, said emitted light being able to trespass the internal wall 8 promoting the growth of the algae.

The glowing liquid 14 can be circulated inside the internal wall 8, so it can transport light from the more illuminated parts of the internal wall 8, for example the top portion in which a light source 15, formed by a surface provided with leds, powers the glowing liquid 14, towards the more dark parts, thus uniformly distributing light among the internal wall 8.

The photobioreactor PBR can also be fitted with pumping means 16, such as a liquid pump, for transferring said glowing liquid within the internal wall 8, for example extracting glowing liquid 14 from the internal wall 8 in the top of the tank 2 and injecting said glowing liquid 14 to the bottom of the internal wall 8.

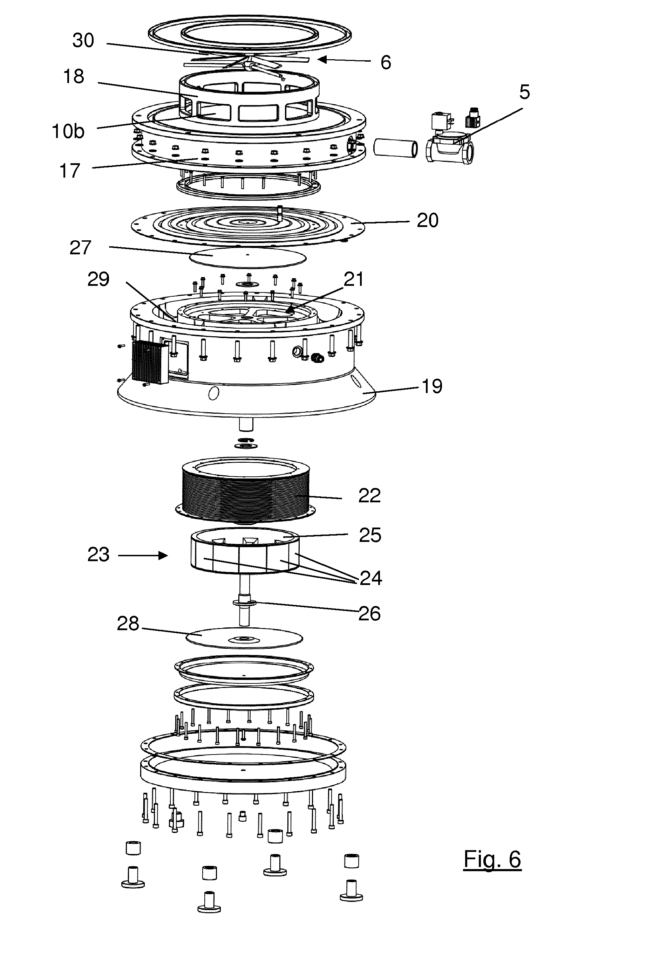

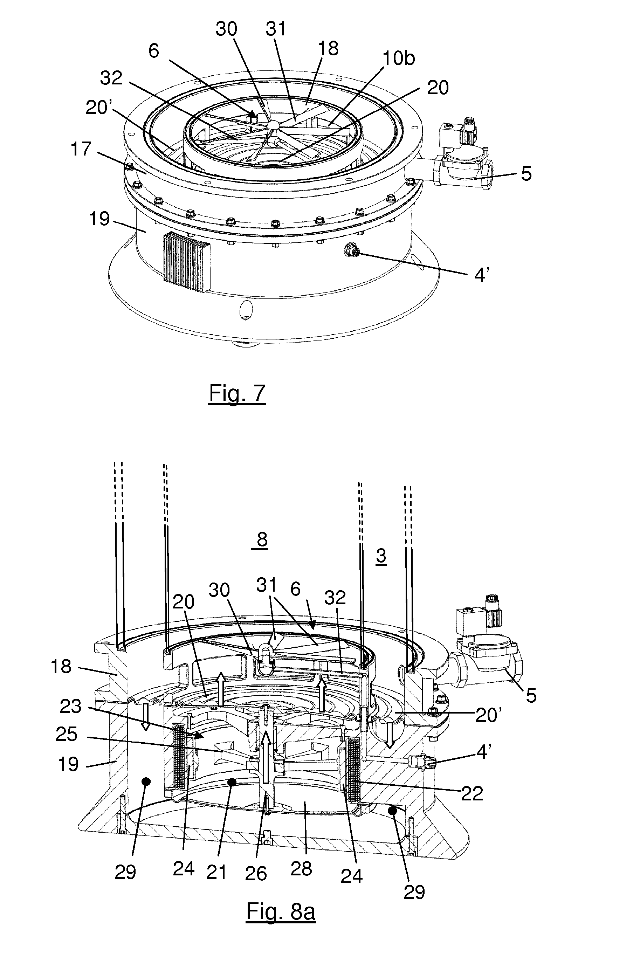

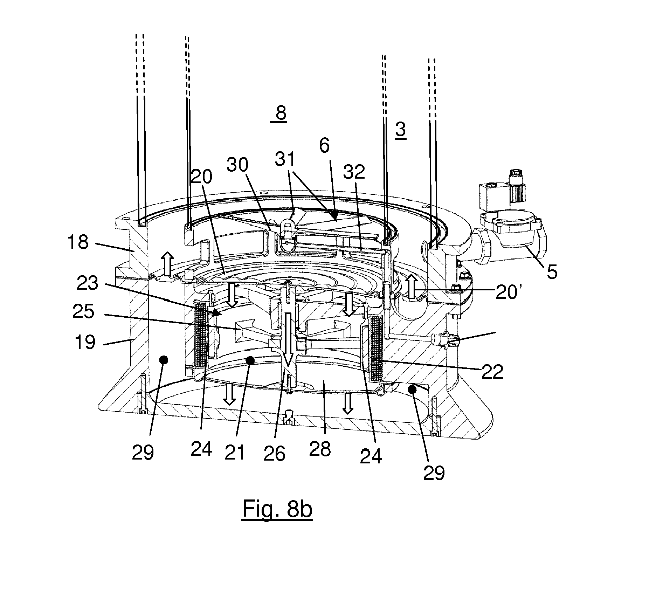

Fig. 6 discloses an exploded view of an embodiment of the base of the photobioreactor PBR, including a variant of the pressure wave pulsation means 11 and a variant of the CO2 applying means 6; Fig. 7 shows the base of Fig. 6 assembled and Figs. 8a and 8b a section view of the assembled base showing both directions of the pulsation waves in both the first and second chambers 9a, 9b. In other variants of the photobioreactor PBR the base and the pressure wave pulsation means 11 and CO2 applying means 6 could even be placed in other parts of the photobioreactor PBR, for example at the top.

As it can be seen, the base of Fig. 6 comprises an external top piece 17 and an internal top piece 18, said internal top piece 18 being provided with the bottom apertures 10b through which the solution circulates, said external top piece 17 and internal top piece 18 are arranged to receive the insertion of the external wall 3 and internal wall 8 respectively. The external top piece 17 has the outlet 5 of the photobioreactor PBR.

The external and internal top pieces 17, 18 are attached to the bottom 19 of the photobioreactor PBR with a flexible membrane 20 placed between the bottom 19 and the internal and external top pieces 17, 18 to transmit pressure waves generated by the pressure wave pulsation means 11 to the first and second chambers 9a, 9b. The bottom 19 also has the CO2 inlet 4' of the photobioreactor PBR that will drive the CO2 towards the CO2 applying means 6.

The bottom 19 comprises a cavity 21 arranged to host a coil 22, winded for example in a static drum 23 as in the depicted embodiment, and a magnetic body 23, provided with magnets 24, being surrounded by said coil 22. The magnetic body 23 is placed in the cavity 21 arranged to perform an up and down movement when the coil 22 is powered. The magnetic body 23 could be a wheel 25 provided with magnets 24 in its ends, said magnets being placed with the same polarity, for example North in the outmost part of the wheel 25, so the wheel 25 is arranged to perform the up and down movement when alternatively a positive and negative current passes through the coil 22.

The magnetic body 23 has a shaft 26 attached to a top membrane 27, so when the magnetic body 23 moves vertically, the shaft 26 transfers the vertical movement to the top membrane 27 and the top membrane 27 to the central part of the flexible membrane 20, generating a pressure wave in the first chamber 9a. Alternatively, the shaft 26 could directly be attached or hit the flexible membrane 20 as previously shown in the pressure wave pulsation means 11 depicted in Fig. 4.

Contrary that in the pressure wave pulsation means 11 depicted in Fig. 4, where the bottom of the shaft is not used to generate pressure waves, in the wave pulsation means 11 of Figs. 6 to 8b the bottom of the shaft 26 is attached to a bottom membrane 28, space between the top and bottom membranes 27, 28 being preferably filled with a technical liquid like oil or a ferrofluid. When the liquid is a ferromagnetic liquid or ferrofluid it also helps to dissipate the heat of the coil, so it works more efficiently, and decreasing resistance and allowing gradual viscosity. A ferrofluid is a stable colloidal suspension of sub-domain magnetic particles in a liquid carrier as known in the state of the art.

Also, the base 19 has peripheral channels 29, also preferably filled with a technical liquid like oil or a ferrofluid, so the bottom membrane 28 is hydraulically connected with an external flexible membrane 20' that in this embodiment is integral with the flexible membrane 20. Said external flexible membrane 20' is arranged to generate and receive vertical pressure waves from the second chamber 9b. With this arrangement simultaneous inverted pressure waves are advantageously created in the first and second chambers 9a, 9b through the flexible membrane 20 and the external flexible membrane 20'.

Advantageously, when the base 19 and the space between the top and bottom membranes 27, 28 is filled with a ferrofluid, and when said top, bottom and flexible membranes 27, 28, 20 have high sensitivity, the changes in polarisation in the coil 22 causing changes in density in the contained ferroliquid following the inducted magnetic field would be enough to generate the pressure waves in the flexible membrane 20, in a similar way as if they were hit mechanically. So the ferrofluid actuated by the coil 22 could be enough to generate the pressure waves in the flexible membrane 20 and the magnetic body 23 provided with a shaft 26 could be removed. When the peripheral channels 29 are also filled with ferrofluid, this ferrofluid not only is hydraulically pushed by the bottom membrane 28, but also the magnetic field of the coil 22 causes and up and down movement in this ferrofluid. In order to enhance the hydraulical connection through peripheral channels 29, deflectors could be placed in the base 19, as a central bottom vortex-like shape cone in the bottom of the base 19.

Then, when the coil 22 is powered with an alternating current, the frequency of said alternating current is the frequency of the pressure wave emitted by the separation membrane 20 and thus generated by the pressure wave pulsation means 11 towards the tank 2. The amplitude of the pressure wave will be proportional to the amount of peak current passing through the coil 22, generating the magnetic field that moves up and down the magnetic body 23.

Advantageously, when the shaft 26 is connected to both the top and bottom membranes 27, 28, the pressure of the liquid placed within the chambers towards the separation membrane 20, applied to both top and bottom membranes resets the magnetic body 23 to its central position, thus saving energy so advantageously no current has to be used for resetting the position of the magnetic body 23. It then allows the coil 22 to be powered just with periodic electrical impulses of different polarities instead of with a continuous alternating signal.

Naturally, the magnetic arrangement disclosed could be replaced by any other magnetic or even mechanic arrangement allowing moving the shaft to transfer the vertical movement to at least one of the membranes.

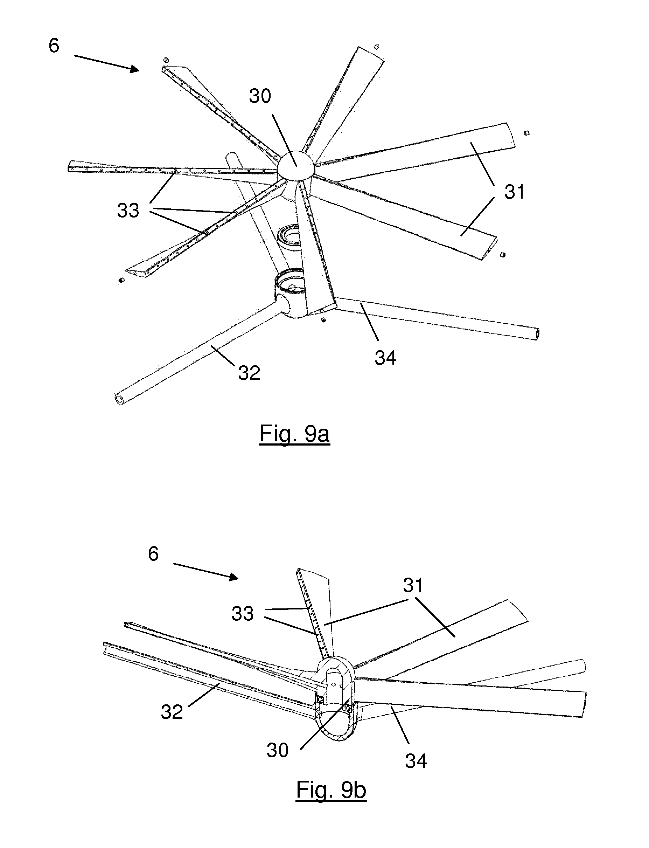

Figs. 9a and 9b discloses the detail of the alternative embodiment of the CO2 applying means 6 shown in Figs. 6 to 8b. This embodiment of the CO2 applying means 6 is a fan 30 provided with blades 31, each blade 31 comprising a CO2 conduct 32 and a number of holes 33 in its upmost edge, arranged to expel CO2 while causing the rotation of the fan 30 in a direction to which the blades 31 causes the upward movement of the liquid contained in the first chamber 9a. CO2 is fed to the fan 30 through at least a hollow tube 32 of the support 34 of the fan 30, connected to the CO2 inlet. The support 34 of the fan 30 is attached to the base in the bottom of the first chamber 9a, the fan 30 being rotatably attached to the support 34 through a bearing joint forcing the CO2 fed though the hollow tube 32 to exit the fan 30 only through the holes 33. Although in the present embodiment only one tube 32 of the support 34 is hollow and feds CO2, in other embodiments more than one tube 32 of the support 34 could be used not only to feed CO2 but also prepared water WPS, nutrition solution NAS or even a mix of them.

## Claims

1. Photobioreactor (PBR) for algae growth, comprising a tank (2) having an external wall (3), an inlet (4) for adding a medium for algae growth, an outlet (5) for extracting algae from the photobioreactor, CO2 applying means (6) and lighting means (7) **characterized in that** it further comprises an internal wall (8), determining a first chamber (9a) and a second chamber (9b) in the tank, said first and second chambers being intercommunicated, such that the medium of the tank can flow between chambers; the CO2 applying means being arranged in said first chamber for bubbling CO2 to the medium.

2. Photobioreactor (PBR) according to the preceding claim, **characterized in that** the internal wall (8) is cylindrical, the first chamber (9a) being surrounded by said internal wall and the second chamber (9b) being determined between the internal wall and the external wall (3).

3. Photobioreactor (PBR) according to any of the preceding claims, **characterized in that** the first chamber (9a) further comprises a pressure wave pulsation means (11), for applying pressure waves that propagate in a vertical direction, said pressure wave pulsation means being placed behind the CO2 applying means (6), such that the pressure waves generated by the pressure wave pulsation means hit the CO2 bubbled by the CO2 applying means.

4. Photobioreactor (PBR) according to any of the preceding claims, **characterized in that** the CO2 applying means (6) is an essentially flat tubular spiral (12) provided with holes for bubbling CO2.

5. Photobioreactor (PBR) according to any of the claims 1 to 3, **characterized in that** the CO2 applying means (6) is a fan (30) provided with holes (33) in the lateral edge of its blades (31) for bubbling CO2.

6. Photobioreactor (PBR) according to any of the claims 3 to 5, **characterized in that** the pressure wave pulsation means (11) generates pressure waves with a frequency up to 100MHz and amplitude up to 80mm.

7. Photobioreactor (PBR) according to the preceding claim, **characterized in that** the pressure wave pulsation means (11) generates pressure waves with a frequency between 60Hz and 80Hz and an amplitude between 60mm and 80mm.

8. Photobioreactor (PBR) according to any of the claims 3 to 7, **characterized in that** the pressure wave pulsation means (11) comprises a flexible membrane (20) placed at the bottom of the first chamber (9a) and actuating means actuating said flexible membrane to transmit pressure waves to said first chamber.

9. Photobioreactor (PBR) according to the preceding claim, **characterized in that** it further comprises a top membrane (27) placed under the flexible membrane (20) and a bottom membrane (28), the gap between the top and bottom membranes being surrounded by a coil (22) and said bottom membrane being hydraulically connected to an external flexible membrane (20') placed at the bottom of the second chamber (9b) and arranged to generate vertical direction pressure waves in said second chamber,

10. Photobioreactor (PBR) according to the preceding claim, **characterized in that** the gap between the top and bottom membranes (27, 28) contains a ferrofluid.

11. Photobioreactor (PBR) according to any of the claims 9 to 10, **characterized in that** the gap between the top and bottom membranes (27, 28) contains a shaft (26) attached to the top and bottom membranes rigidly attached to a magnetic body (23) performing an up and down movement when the coil (22) is powered with an alternate current.

12. Photobioreactor (PBR) according to any of the preceding claims, **characterized in that** the internal wall (8) is hollow, transparent and embeds the lighting means, the lighting means embedded in the internal wall (8) comprising a glowing liquid (14) and the lighting means further comprising a light source (15) powering the glowing liquid and pumping means (16) for moving said glowing liquid within the internal wall (8).

13. Use of cavitation activated water for growing algae in a photobioreactor (PBR) according to any of the preceding claims.

14. System (1) for algae growth comprising:
(a) a water preparation unit (WPS) comprising:
(1) means for filtering said water;
(2) means for sterilizing said water; and
(3) means for carbonating said water;
(b) a nutrition algae system (NAS) comprising:
(1) means for mixing Ca(OH)2 with CO2 obtaining a first mix comprising Ca(HCO3)2;
(2) means for mixing said first mix with a nitrogen oxide (N2O, NO, NO2) and/or with a sulphur oxide (SO2) to obtain a second mix respectively comprising CaSO4 and/or CaNO4;
(3) means for mixing said second mix with sewage and yeast obtaining prepared nutrition solution
the system being **characterized in that** it further comprises one or more photobioreactors (PBR) according to any of the preceding claims; said one or more photobioreactors comprising at least an algae strain to be grown; the one or more photobioreactors being connected to the water preparation unit for providing prepared water to said one or more photobioreactors, and also being connected to the nutrition algae system for providing nutrition solution to the said one or more photobioreactors;
the system being further **characterized in that** the water preparation unit comprises means for activating said water through cavitation to obtain the prepared water.

15. Process for growing algae comprising the steps of:
(a) preparing water in a water preparation unit (WPS) through the sequential steps of:
(1) filtering said water,
(2) sterilizing said water,
(3) carbonating said water with the addition of CO2;
(b) preparing a nutrition solution in a nutrition algae system (NAS) through the sequential steps of:
(1) mixing Ca(OH)2 with CO2 obtaining a first mix comprising Ca(HCO3)2;
(2) mixing said first mix with a nitrogen oxide (N2O, NO, NO2) and/or with a sulphur oxide (SO2) to obtain a second mix respectively comprising CaSO4 and/or CaNO4;
(3) mixing said second mix with sewage and yeast obtaining prepared nutrition solution;
**characterised in that** the process further comprises the step of
(c) adding an algae strain to one or more photobioreactors according to claims 1 to 13, together with prepared water from the water preparation unit and nutrition solution from the prepared nutrition solution, for growing the algae population and applying light and CO2 in said photobioreactor for growing the algae population,
and also **in that** preparing water in the water preparation unit comprises the additional step of activating said water through cavitation.
